(19) **Europäisches Patentamt / European Patent Office / Office européen des brevets**

(11) **EP 1 057 525 B1**

(12) **EUROPÄISCHE PATENTSCHRIFT**

(45) Veröffentlichungstag und Bekanntmachung des Hinweises auf die Patenterteilung:
**05.03.2008 Patentblatt 2008/10**

(51) Int Cl.:
**B01J 8/06** (2006.01) **C07C 69/01** (2006.01)

(21) Anmeldenummer: **00109784.9**

(22) Anmeldetag: **09.05.2000**

(54) **Verfahren zur katalytischen Vinylierung von Carbonsäuren**

Process to perform catalysed vinylation of carboxylic acids

Procédé pour réaliser des réactions catalysées de vinylation d'acides carboxyliques

(84) Benannte Vertragsstaaten:
**AT BE CH CY DE DK ES FI FR GB GR IE IT LI LU MC NL PT SE**

(30) Priorität: **02.06.1999 DE 19925385**

(43) Veröffentlichungstag der Anmeldung:
**06.12.2000 Patentblatt 2000/49**

(73) Patentinhaber: **Evonik Oxeno GmbH**
**45772 Marl (DE)**

(72) Erfinder:
• **Wiese, Klaus-Diether, Dr.**
**45721 Haltern (DE)**

• **Olbrich, Paul, Dr.**
**45721 Haltern (DE)**

(74) Vertreter: **Hirsch, Hans-Ludwig et al**
**Evonik Degussa GmbH**
**DG-IPM-PAT**
**Bau 1042 / PB 15**
**Paul-Baumann-Strasse 1**
**45764 Marl (DE)**

(56) Entgegenhaltungen:
**EP-A- 1 057 524**      **WO-A-98/16494**
**DE-B- 1 210 810**      **DE-B- 1 238 010**

**Beschreibung**

Die Erfindung betrifft ein Verfahren zur katalytischen Herstellung von Vinylestern durch Reaktion von Carbonsäuren mit Acetyl in einem Rohrreaktor.

Vinylester sind Bausteine für die Herstellung von Copolymeren. Von besonderer technischer Bedeutung sind Vinylester von tertiären $C_{10}$-Säuren, ferner von tertiären $C_9$-Säuren. Diese Vinylester können mit Vinylacetat copolymerisiert werden und dienen dann als Komponente für Anstrichmittel. Bedeutung haben auch die Vinylester der 2-Ethylhexansäure, der Isononansäure, aber auch der Laurin- und Stearinsäure, zum Beispiel bei der Herstellung von Klebstoffen.

Die Umsetzung von Carbonsäuren mit Acetylen zu Vinylestern ist lange bekannt (z. B. G. Hübner, Fette, Seifen, Anstrichm. 68, 290 (1966)), eine Übersicht über gängige Prozesse ist zum Beispiel in Ullman's Encyclopedia of Industrial Chemistry, Fifth Completely Revised Edition, Volume A27, 430-434 nachzulesen. Die Reaktion von Acetylen und Vinylester kann gemäß der Formel

$$R\text{-}COOH + C_2H_2 \text{ ----------> } R\text{-}COO\text{-}CH{=}CH_2$$

beschrieben werden. Als Katalysator wird meist das Zink-Salz der umzusetzenden Carbonsäure, in überschüssiger Carbonsäure gelöst, eingesetzt (DE 1238010, DE 1210810). In einem anderen Verfahren wird in einem paraffinischen Lösungsmittel gearbeitet, das höher siedet als die anzuwendende Reaktionstemperatur (z.B. Paraffinöle), wobei die Konzentration an Carbonsäure unter 5 Gew.-% und der Zink-Gehalt bei 0,5 bis 5 Gew.-% bezogen auf die Reaktionslösung zu halten ist. Die angewendeten Temperaturen liegen in der Regel über 200 °C, typisch bei 220 bis 295 °C. Es muß mit einem erheblichen molaren Überschuß an Acetylen bezogen auf die zu vinylierende Carbonsäure gearbeitet werden, typisch sind Werte von 5 - 20 Mol Acetylen pro Mol umzusetzender Carbonsäure. Dies bedingt einen hohen Acetylenkreislauf, wobei der Vinylester und nicht umgesetzte Carbonsäure entsprechend ihrem Partialdampfdruck mit dem Abgas ausgetragen, anschließend auskondensiert und durch Destillation von einander getrennt werden.

Die Bildung von leichtsiedenden Nebenprodukten wie Acetaldehyd kann zur Verharzung des Katalysators führen. Weiterhin kann die Bildung von Hochsiedern durch Polymerisation des gebildeten Vinylesters sowie die Bildung von Carbonsäureanhydriden, die nicht mehr an der Reaktion teilnehmen, zu Umsatzeinbußen führen. Dies ist nur durch Ausschleusung eines Teils der Reaktionslösung bzw. der Zinksalzschmelze zu korrigieren, was wiederum die Zuführung von Frischkatalysator erfordert. Den Beispielen in DE 1 237 557 ist zu entnehmen, daß bei entsprechend hohem molaren Überschuß von Acetylen zu Carbonsäure von 5:1 bis 20:1 Selektivitäten von 74,3 bis maximal 96,1 % erreicht werden. Dies entspricht einer Nebenproduktbildung von 3,9 % bis 25,7 %.

Technisch gesehen handelt es sich bei der Vinylesterherstellung um eine Zweiphasenreaktion, wobei das Acetylen die Gasphase und die Katalysatorphase, die umzusetzende Carbonsäure, der Vinylester und gegebenenfalls ein inertes Lösungsmittel die flüssige Phase bilden. Zur Erreichung hoher Umsätze sind deshalb Maßnahmen zu treffen, um die Phasen möglichst gut mit einander in Kontakt zu bringen. Üblicherweise werden derartige Gas-Flüssig-Reaktionen in Blasensäulen oder gerührten Reaktoren durchgeführt. Auch Rieselreaktoren werden in der Literatur erwähnt. Hohe Drücke sind speziell bei Reaktionen mit Acetylen nicht erwünscht, da Acetylen zum Selbstzerfall neigt und bei reinem Acetylen ein Überdruck von 300 mbar (Absolutdruck 1,3 bar) nicht überschritten werden darf Dies engt den Spielraum bei der Reaktorauswahl erheblich ein. Für die Vinylierung sind somit nur kurze Blasensäulen oder gerührte Reaktoren geringer Höhe einsetzbar, da sonst allein schon der statische Druck der Flüssigkeit einen höheren Acetylenvordruck als 300 mbar erfordert. Darüber hinaus erfordern die eventuell notwendigen hohen Acetylenkreisläufe eine dauernde Neuverdichtung des Acetylens.

Eine Alternative ist der Einsatz von höher verdichtetem Acetylen, wobei die Zerfallsgrenze durch Zugabe großer Anteile von Inertgasen wie Stickstoff gesenkt werden muß. Diese Maßnahme fuhrt zu Problemen bei der Ausschleusung des Restgases und ist auch sicherheitstechnisch nicht unproblematisch.

Es wurden daher andere Verfahren entwickelt, die diese Nachteile vermeiden oder mindern. So werden zur Vinylesterherstellung in EP 0 622 352 Platinmetallkomplexe (insbesondere Ruthenium) als Katalysatoren eingesetzt, wodurch die Reaktionstemperatur deutlich gesenkt werden konnte. Doch wird auch hier bevorzugt bei einem Molverhältnis Acetylen/Carbonsäure von 1,5:1 bis 10:1 und bei Acetylendrücken von 15 bis 20 bar gearbeitet, um ausreichende Raum-Zeit-Ausbeuten zu erreichen. Der hohe Preis für Edelmetallverbindungen erzwingt eine vollständige Kreislaufführung des Katalysators, ohne dessen Zersetzung oder Verluste durch unbeabsichtigtes Ausschleusen. Ein weiteres Beispiel für Ruthenium-katalysierte Vinylesterherstellung liefert US 5430179.

Ein gänzlich anderes Verfahren zur Herstellung von Vinylestern bietet die Umvinylierung. Hierbei wird in Anwesenheit von Edelmetallkatalysatoren wie zum Beispiel $Li_2PdCl_4$ ein technisch leicht zugänglicher Vinylester wie Vinylacetat mit der zu vinylierenden Säure umgesetzt, wobei ein Gleichgewichtsgemisch aus Vinylacetat, Essigsäure, der umzusetzenden Carbonsäure und ihrem Vinylester entsteht. Hier sind Schwierigkeiten und großer Aufwand bei der Auftrennung der Gleichgewichtsgemische, der Preis von Vinylacetat und der Anfall an verunreinigter Essigsäure als Probleme zu nennen.

Unter Abwägung aller Vor- und Nachteile ist die Vinylierung von Carbonsäuren mit Acetylen unter Zinksalzkatalyse, d. h. eine Mehrphasenreaktion, das wirtschaftlichste und großtechnisch einfachste Verfahren.

Unter Mehrphasenreaktionen werden im folgenden Reaktionen verstanden, die unter Beteiligung von zwei oder mehr nicht oder nur teilweise mischbaren fluiden Phasen ablaufen. Dies betrifft z. B. Reaktionen zwischen einer Gas- und einer Flüssigphase (gl) wie bei der Reaktion von Acetylen mit einer Carbonsäure, aber auch Reaktionen zwischen zwei Flüssigphasen, die nicht mischbar sind oder unter den jeweiligen Reaktionsbedingungen eine Mischungslücke aufweisen (ll) und Reaktionen, an denen sowohl zwei flüssige nicht oder nur teilweise mischbare Phasen sowie eine Gasphase beteiligt sind (gll).

Beispiele für technisch wichtige Gas-Flüssig-Reaktionen (gl) sind neben der betrachteten Reaktion von Acetylen mit Carbonsäuren zum Beispiel Hydrierungen mit homogen gelösten Katalysatoren, Oxidationen mit Luft oder Sauerstoff und die Hydroformylierung von Olefinen.

Mehrphasenreaktionen sind allgemein mit einer Reihe von Problemen verbunden, die ihre technische Ausführung wesentlich schwieriger als einfache homogene Reaktionen macht. Im folgenden sind einige typische Probleme beschrieben:

In allen Fällen müssen die Stoffe möglichst innig miteinander in Kontakt gebracht werden, um das Problem des Stoffüberganges zu minimieren: es muß eine möglichst große Stoffübergangsfläche $a_s$ zwischen den Phasen erzeugt werden. Andererseits müssen die Phasen nach erfolgter Reaktion wieder leicht getrennt werden können: zu starke Vermischung kann hier zu Problemen führen. Bei Anwesenheit zweier flüssiger Phasen kann es zur Emulsionsbildung kommen, bei Gas-Flüssig-Verfahren zum Schäumen. Bei den erwähnten 3-Phasenverfahren können sogar alle Probleme gleichzeitig auftreten.

Neben einer hohen Stoffübergangsfläche $a_S$ sollte in allen Mehrphasenreaktionen ein möglichst hoher Stoffübergangskoeffizient $k_1$ erreicht werden. Insgesamt sollte der sogenannte KLA-Wert, d.h. das Produkt aus $k_1$ und $a_S$ in der Stoffübergangsgleichung

$$j \quad = k_l * a_S * (C^* - C)$$

mit

- $j$    [Mol/s] der durch die Phasengrenzfläche hindurchtretende Molenstrom der reagierenden Komponente (z. B. Eintritt von Acetylen in die Katalysatorphase),
- $k_1$    [m/s] Stoffübergangskoeffizient,
- $a_S$    [m$^2$] Phasengrenzfläche im Reaktor,
- $C^*$    [Mol/m$^3$] kennzeichnet die maximale Löslichkeit des Edukts in der zweiten Phase (z. B. Acetylen in der Katalysatorphase) und
- $C$    [Mol/m$^3$] tatsächliche Konzentration des Edukts, die wiederum mit der Reaktionsgeschwindigkeit gekoppelt ist,

maximal sein.

Im Hinblick auf die voranstehenden Darlegungen besteht ein Bedarf für ein Verfahren zur Durchführung von Mehrphasenreaktionen, daß die vorstehend genannten Nachteile vermeidet und sich zudem auf einfache Weise realisieren läßt.

Aufgabe der vorliegenden Erfindung ist somit die Entwicklung eines Verfahrens zur Durchführung von Mehrphasenreaktionen, das sich für die Herstellung von Vinylestern durch katalysierte Umsetzung von Carbonsäuren mit Acetylen eignet.

Technisch sollte das neue Verfahren folgende Ansprüche an ein Mehrphasenverfahren erfüllen:

- Erzeugung eines hohen und stabilen Stoffübergangs zwischen den beteiligten Phasen
- Einfach ausführbar, möglichst mit üblichen technischen Apparaten
- Einfache und sichere Wärmeabfuhr
- Hohe Betriebssicherheit
- Einfache und sichere Maßstabsübertragung

In Bezug auf die durchzuführende Reaktion von Acetylen mit Carbonsäure kommen speziell hinzu:

- Hohe Selektivität, Vermeidung insbesondere hochsiedender Nebenprodukte
- Keine oder nur geringfügige Katalysatorausschleusung

- Hohe Raum-Zeit-Ausbeute, kleine Reaktoren
- Hoher Acetylenumsatz im geraden Durchgang, Vermeidung von Kreislaufführung von Acetylen
- Einfacher und sicherer Umgang mit Acetylen
- Hohe Produktreinheit

Mit dem erfindungsgemäßen Verfahren wurde ein überraschend einfaches Verfahren zur Durchführung von Mehrphasenreaktionen gefunden, das in einem Rohrreaktor - gegebenenfalls gefüllt mit Füllkörpern oder Einbauten - durchgeführt werden kann und zur Vinylierung von Carbonsäuren mit Acetylen unter hohen Raum-Zeit-Ausbeuten und Selektivitäten geeignet ist.

Gegenstand der vorliegenden Erfindung ist daher ein Verfahren zur katalytischen Durchführung von Mehrphasenreaktionen in einem Rohrreaktor, wobei der Katalysator in der kontinuierlichen Phase und mindestens ein Edukt in einer dispergierten Phase enthalten ist und der Belastungsfaktor B des Reaktors gleich oder größer 0,8 ist.

Durch die Mehrphasenreaktion werden Carbonsäuren mit Acetylen zu den entsprechenden Vinylestern umgesetzt.

Das erfindungsgemäße Verfahren eignet sich zur Vinylierung von Carbonsäuren; diese können 2 bis 16, bevorzugt 4 bis 13 Kohlenstoffatome enthalten. Besonders bevorzugt werden Pivalinsäure ($C_5$), 2-Ethylhexancarbonsäure ($C_8$), Neononansäure ($C_9$), Decansäure ($C_{10}$), Undecansäure ($C_{11}$), Laurinsäure ($C_{12}$) oder Tridecansäure ($C_{13}$) eingesetzt.

Wie bereits ausgeführt, kann der Rohrreaktor Füllkörper oder Einbauten enthalten. Füllkörper im Sinne der vorliegenden Erfindung sind beispielsweise: Raschigringe, Sättel, Pallringe, Telleretten, Maschendrahtringe, Maschendrahtgewebe. Beispiele für Einbauten sind Filterplatten, Strombrecher, Kolonnenböden, Lochbleche oder sonstige Mischvorrichtungen. Als Einbauten im Sinne der vorliegenden Erfindung sind aber auch mehrere enge, parallel geschaltete Rohre denkbar, es resultiert also ein Viellrohrreaktor. Besonders bevorzugt sind strukturierte Mischerpackungen oder Demisterpackungen.

Entscheidende Bedeutung hat im erfindungsgemäßen Verfahren die Einhaltung bzw. Überschreitung einer minimalen Querschnittsbelastung des Rohrreaktors. Bei Aufwärtsbetrieb des Reaktors (Strömungsrichtung von unten nach oben) sollte der Flutpunkt überschritten werden. Der Reaktor wird also oberhalb des Punktes betrieben, bei dem man üblicherweise Blasensäulen betreibt. Bei Abwärtsbetrieb (Strömungsrichtung von oben nach unten) ist die Querschnittsbelastung so einzustellen, daß der Reaktor vollständig geflutet ist. Man arbeitet somit oberhalb des Punktes, bei dem man noch von einer Rieselphase (trickle bed) sprechen kann.

Zur genaueren Festlegung der minimal einzuhaltenden Belastung des Reaktors wird der Belastungsfaktor B des Rohrreaktors als ein dimensionsloser Druckverlust mit

$$B = PD/PS$$

berechnet, wobei PD [Pa/m] ein längenbezogener Druckverlust über den Reaktor unter Betriebsbedingungen und PS [Pa/m] eine Rechengröße mit der Dimension eines längenbezogenen Druckes, definiert als Verhältnis von Massenstrom M [kg/s] aller Komponenten im Reaktor zum Volumenstrom V [m³/s] aller Komponenten unter Betriebsbedingungen, multipliziert mit g = 9,81 m/s², d. h. PS = (M/V)*g, bedeuten.

Anschaulich wäre PS der statische Druck pro Meter eines Mehrphasengemisches in einem senkrecht stehenden Rohr, wenn alle Phasen mit gleicher Geschwindigkeit strömen würden. PS ist eine reine Rechengröße, die sich aus den dem Reaktor zugeführten Mengenströmen ergibt und die unabhängig von der Strömungsrichtung des Reaktors, der Strömungsgeschwindigkeit aller Phasen oder des Flutzustandes des Reaktors angegeben werden kann.

Der Druckverlust PD [Pa/m] wird als Rechengröße, um die Prozeßbedingungen festzulegen, verwendet und kann nach den gängigen Methoden für Ein- bzw. Mehrphasenströmungen berechnet werden. Gängige Verfahren zur Berechnung des Druckverlustes PD in Rohren, Einbauten oder Füllkörperschüttungen usw. können z. B. im VDI-Wärmeatlas 7. Erweiterte Auflage, VDI-Verlag GmbH, Düsseldorf 1994, Abschnitte La1 bis Lgb7, sowie im Standardwerk Heinz Brauer, Grundlagen der Einphasen- und Mehrphasenströmungen, Verlag Sauerländer, Aarau und Frankfurt am Main, 1971, nachgeschlagen werden.

Der Druckverlust PD ist bei der Einphasenströmung durch ein leeres Rohr durch

$$PD = Cw*\rho/2*w^2/D$$

mit

ρ     $[kg/m^3]$ Dichte des strömenden Mediums unter Betriebsbedingungen,

w     [m/s] Strömungsgeschwindigkeit (Volumenstrom/Querschnittsfläche),

D     [m] Rohrdurchmesser und

Cw     [-] Widerstandsbeiwert des durchströmten Rohres

gegeben.

Bei einer Strömung durch Füllkörper, Schüttungen oder Einbauten ist die Geschwindigkeit w durch die Effektivgeschwindigkeit (w/ψ) sowie der Rohrdurchmesser D durch den hydraulischen Kanaldurchmesser $d_H$ der Füllkörper oder Einbauten zu ersetzen, so daß gilt:

$$PD = Cw * \rho/2 * (w/\psi)^2 * 1/d_H$$

mit

$d_H$     [m] Hydraulischer Kanaldurchmesser

ψ     [-] Leerrohranteil

Cw     [-] Widerstandsbeiwert des durchströmten Apparates mit Füllung

Die füllkörperspezifischen Daten $d_H$ und ψ sind häufig Bestandteil der Lieferspezifikationen von Füllkörpern. Für eine Reihe von Füllkörpern sind Daten im oben erwähnten VDI-Wärmeatlas angegeben.

Der Leerrohranteil ψ kann auch experimentell bestimmt werden, indem man zum Beispiel den Reaktor vor und nach Befüllung mit den Füllkörpern auslitert. Der hydraulische Kanaldurchmesser wiederum kann, wenn er nicht bekannt ist, aus der spezifischen Oberfläche F $[m^2/m^3]$ der Füllkörper oder Einbauten (in der Regel bekannt oder experimentell bestimmbar) nach der einfachen Beziehung

$$d_H = 4\psi/F.$$

berechnet werden.

Der Widerstandsbeiwert von Rohren, Einbauten und Füllkörpern wird in der Regel in Abhängigkeit von der Reynoldszahl Re beschrieben, die Auskunft über den Strömungszustand unter den gewählten Bedingungen gibt. Bei Füllkörpern, Einbauten usw. ist fast immer folgende Beziehung anwendbar:

$$Cw = K_1/Re^n + K_2/Re^m$$

wobei häufig n = 1, m = 0 (Ansatz nach S. Ergun, Chem. Engng. Progr. 48, (1948), 89), oder n = 1, m = 0,1 (Ansatz nach Brauer et al.) verwendet wird. $K_1$, $K_2$ sind füllkörperspezifische Konstanten, die aus Lieferdaten oder aus der Literatur bekannt sind (Beispiele sind im VDI-Wärmeatlas und bei Brauer et al. zu finden). Sie können aber auch experimentell bestimmt werden, indem man den Rohrreaktor mit Füllkörpern mit einer Flüssigkeit unter verschiedenen Geschwindigkeiten betreibt und aus den bekannten Daten und dem gemessenen Druckverlust Cw in Abhängigkeit von Re bestimmt.

Die dimensionslose Reynoldszahl Re schließlich ist definiert als

Re = w*(ρ/η)*D für Leerrohre bzw.

Re = (w/ψ) * (ρ/η)*$d_H$ für Rohre mit Einbauten oder Füllkörpern. ρ [Pa*s] bezeichnet jeweils die Viskosität und ρ $[kg/m^3]$ die Dichte des strömenden Mediums.

Der Druckverlust bei Zweiphasenströmungen (gas-flüssig für Acetylen/Carbonsäure) steigt überproportional an. Meist wird nach Lockhart-Martinelli (in Brauer et al.) der Druckverlust der Zweiphasenströmung $P_{lg}$ auf den Druckverlust einer der beiden Phasen bezogen, zum Beispiel auf den Druckverlust der reinen strömenden flüssigen Phase $P_l$, und in Beziehung zu dem Verhältnis des Druckverlustes der beiden als allein strömend gedachten Phasen $P_l$ und $P_g$ gesetzt.

Zur Berechnung von Druckverlusten in Zweiphasenströmungen werden häufig dimensionslose Drücke nach $\phi^2 = P_{lg}/P_1$ und $X^2 = P_l/P_g$ eingesetzt. Der weitere Zusammenhang $\phi^2$ = Funktion($X^2$) ist vielfach untersucht. Beispiele finden sich in folgenden Literaturstellen:

Y. Sato, T.Hirose, F. Takahashi, M. Toda: "Pressure Loss and Liquid Hold Up in Packed Bed Reactor with Cocurrent Gas-Liquid Down Flow"; J. Chem. Chem. Eng. Of Japan, Vol 6 (Nr. 2), 1973, 147-152;

D. Sweeney: "A Correlation for Pressure Drop in Two-Phase Concurrent Flow in Packed Beds"; AIChE-Journal, Vol. 13, 7/1967, 663-669;

V. W. Weekman, J. E. Myers: "Fluid-Flow Characteristics of Concurrent Gas-Liquid Flow in Packed Beds"; AIChE-Journal, Vol 10 (Nr. 6), 11/1964, 951-957;

R. P. Larkins, R. P. White, D. W. Jeffrey: "Two-Phase Concurrent Flow in Packed Beds"; AIChE-Journal, Vol 7 (Nr. 2), 6/1961, 231-239 oder

N. Midoux, M. Favier, J.- C. Charpentier: " Flow Pattern, Pressure Loss and Liquid Holdup Data in Gas- Liquid Down-Flow Packed Beds with Foaming and Nonfoaming Liquids"; J. Chem. Eng. Of Japan, Vol 9 (Nr. 5), 1976, 350-356.

Häufig wird für die Berechnung der von Midoux vorgeschlagene Zusammenhang, der für viele Gas-Flüssig-Systeme überprüft wurde, benutzt. Bei nichtschäumenden Systemen ist beispielsweise

$$\phi^2 = 1 + 1/X + 1,14/X^{0,54}$$

Dieser nach Lockart-Martinelli benannte Zusammenhang ist in vielen Werken graphisch dargestellt, detaillierte Abhandlungen hierüber finden sich in vielen Lehrbüchern der Verfahrenstechnik und Veröffentlichungen, so auch bei Brauer et al..

Der Druckverlust der Mehrphasenströmung $P_{lg}$ ergibt sich aus dem experimentell bestimmten oder wie oben erläutert abgeschätzten Druckverlust der reinen strömenden Flüssigphase $P_1$ dann mit

$$P_{lg} = \phi^2 * P_l$$

und es gilt mit der Reaktorlänge L [m]

$$PD = P_{lg}/L.$$

Der Druckverlust einer Mehrphasenströmung ist somit durch übliche Mittel der chemischen Verfahrenstechnik berechenbar. Analoges gilt für den zuvor definierten dimensionslosen Druckverlust B, d. h. den Belastungsfaktor des Mehrphasenreaktors.

Die Größe des dimensionslosen Belastungsfaktors B stellt eine notwendige Grundbedingung des erfindungsgemäßen Verfahrens dar; B sollte größer oder gleich 0,8, bevorzugt größer oder gleich 0,9 oder besonders bevorzugt größer oder gleich 1 sein.

Im Bereich B größer oder gleich 0,8 beginnt ein von oben nach unten betriebener Reaktor zu fluten. Es sei ausdrücklich darauf hingewiesen, daß bei Einhaltung dieser Bedingungen die Vorteile des erfindungsgemäßen Verfahrens auch dann erzielt werden, wenn der Reaktor von unten nach oben oder in anderer Richtung betrieben wird.

Höhere Querschnittsbelastungen des Reaktors (B >> 1), erkennbar am steigenden Differenzdruck über den Reaktor, sind jederzeit möglich und sogar erwünscht, solange die steigenden Raum-Zeit-Ausbeuten den gleichermaßen steigenden Energieverbrauch rechtfertigen. Eine Obergrenze ist daher nur durch praktische Überlegungen wie Energieverbrauch oder Schwierigkeiten bei der Trennung der Phasen nach erfolgter Reaktion gegeben.

Es ist somit ersichtlich, daß neben den Volumenströmen der einzelnen Phasen bzw. den hieraus abgeleiteten Leerrohrgeschwindigkeiten $w = V/(\pi D^2/4)$ die Apparateabmessungen des Reaktors (Länge L, Durchmesser D) sowie insbesondere die Daten der verwendeten Füllkörper (hydraulischer Durchmesser $d_H$, Leerrohranteil $\Psi$) eine wichtige Rolle spielen. Über die richtige Wahl dieser Parameter kann das Verfahren unschwer an die unterschiedlichsten Erfordernisse angepaßt werden, wichtig ist nur die Einhaltung der Forderung B >= 0,8, bevorzugt B >= 0,9 und besonders bevorzugt B >=1.

Bei einer langsamen Reaktion wird man beispielsweise den hydraulischen Durchmesser der Füllkörper klein bzw. ihre spezifische Oberfläche groß wählen, so daß die geforderten Bedingungen für B schon bei kleinen Strömungsgeschwindigkeiten erreicht werden. Man erhält auf diese Weise ausreichende Verweilzeiten über die Länge eines technisch vernünftig dimensionierten Reaktors. Bei sehr schnellen Reaktionen empfiehlt sich eine umgekehrte Verfahrensweise.

Ein weiteres Kriterium bei der Durchführung des erfindungsgemäßen Verfahrens ist das Verhältnis des Massenstroms der flüssigen, den Katalysator enthaltenden Phase $M_1$ zu dem Massenstrom der oder den dispersen Phasen $M_2$. Im Fall der Vinylierung von Carbonsäuren ist der Massenstrom der Katalysatorphase $M_1$ wesentlich größer als der Massenstrom der dispersen Phase, d. h. der Acetylenphase $M_2$. Im erfindungsgemäßen Verfahren kann das Massenverhältnis $M_1/M_2$ der kontinuierlichen Phase ($M_1$) zu den dispersen Phasen ($M_2$) größer 2 sein, vorzugsweise gilt $M_1/M_2$ > 10. Strömungsverhältnisse mit $M_1/M_2$ > 100 sind durchaus möglich und häufig sogar vorteilhaft. Unter der Bedingung $M_1/M_2$ > 2 ist die Katalysatorphase die kontinuierliche Phase, während die disperse Phase in feine Blasen bzw. in feine Tropfen zerteilt wird. Im erfindungsgemäßen Verfahren ist es möglich, daß mindestens ein Edukt durch die von der kontinuierlichen Phase in den Rohrreaktor eingebrachte Energie dispergiert wird. Dies führt zu einer Verteilung zumindest eines Edukts in Blasen oder Tropfen innerhalb der kontinuierlichen Katalysatorphase.

Auch dies läßt sich mit üblichen ingenieurtechnischen Mitteln abschätzen. Es eignen sich hierfür Ansätze mit dimensionslosen Kennzahlen wie

$$d_S/d_H = k * Re_{gl}^{m} * We_{gl}^{n}$$

mit

$d_S$      Durchmesser der Tropfen bzw Blasen nach Sauter (in Brauer et al.)
$d_H$      hydraulischer Füllkörperdurchmesser,
$Re_{gl}$      Reynoldszahl der Mehrphasenströmung = $W_{gl} * (\rho_l/\eta_l) * (d_H/\Psi)$,
$We_{gl}$      Weberzahl der Mehrphasenströmung = $W_{gl} * (\rho_l/\sigma_{gl}) * (d_H/\Psi^2)$,
k, m, n      empirische Konstanten (bekannt oder durch Versuche zu ermitteln),
w      Leerrohrgeschwindigkeiten [m/s] = $V/(\pi D^2/4)$,
V      Volumenstrom unter Betriebsbedingungen [$m^3/s$],
$\rho$      Dichte unter Betriebsbedingungen [$kg/m^3$],
$\eta$      Viskosität unter Betriebsbedingungen [Pa*s] und
$\gamma$      Grenzflächenspannung unter Betriebsbedingungen [N/m]

und den Indices l (Flüssigphase), g (Gasphase) und gl (Gas/Flüssig-Zweiphasenströmung)

Bei strukturierten Packungen wie Sulzer-SMV oder engen Rohren als Einbauten scheint plausibel, daß ein berechneter Blasen- bzw. Tropfendurchmesser $d_s$ größer als der Kanaldurchmesser nicht sinnvoll ist. Dies gilt aber nicht für durchlässige Packungen und Füllkörper wie beispielsweise Maschendrahtringe oder Maschendrahtgewebe (sogenannte Demisterpackungen oder Tropfenabscheider). Im erfindungsgemäßen Verfahren können berechnete Tropfendurchmesser verwendet werden, die mindestens gleich oder kleiner als der hydraulische Kanaldurchmesser sind:

$$d_S/d_H <= 1, \text{ vorzugsweise} < 0{,}9.$$

Aus dem berechneten Tropfendurchmesser läßt sich schließlich eine Stofübergangsfläche nach

$$A_S = 6\varphi_g d_S \ [m^2/m^3]$$

berechnen.

Für den Phasenanteil $\varphi_g$ der dispersen Phase (im Falle der Vinylierung ist die Gasphase bzw. das Acetylen dispergiert), kann mit den Leerrohrgeschwindigkeiten der Phasen

$$\varphi_g \sim w_g/w_{gl}$$

gesetzt werden.

Die Verweilzeit $\tau$ der den Reaktor durchströmenden Phasen läßt sich angenähert nach $\tau \sim L*\psi/w_{lg}$ berechnen. Die Verweilzeit $\tau$ beträgt bei dem erfindungsgemäßen Verfahren in der Regel weit unter einer Stunde und kann im Minutenbereich oder sogar noch darunter liegen. Dennoch werden bei dieser völlig ungewöhnlichen Fahrweise - hoher Katalysatordurchsatz im Reaktor, vergleichsweise sehr geringer Anteil an Edukt an der Reaktionsmasse, dadurch bedingt wiederum sehr kurze Verweilzeit - bei vielen Mehrphasenreaktionen überraschend hohe Raum-Zeit-Ausbeuten erzielt. Alternativ kann bei gleichen Raum-Zeit-Ausbeuten bei deutlich tieferen Temperaturen als üblich gearbeitet werden, da die Steigerung der Reaktionsgeschwindigkeit, was zum Beispiel die Minimierung von Folgereaktionen und damit verbesserte Selektivität nach sich ziehen kann, dies wirtschaftlich zuläßt.

Das erfindungsgemäße Verfahren kann sehr flexibel den unterschiedlichsten Anforderungen angepaßt werden. Für spezielle Anforderungen bieten sich folgende Ausführungsformen des erfindungsgemäßen Verfahrens an:

Erfordert der Einsatzzweck eine sehr lange Durchmischungszone oder sind Ruhezonen z. B. zur Abnahme von Stoffströmen erforderlich, so bietet sich eine kaskadierende Anordnung von Rohrreaktoren mit Einbauten oder Füllkörpern an.

Eine Kaskadierung von Rohrreaktoren oder die alternative Anordnung von gepackten und leeren Rohrabschnitten ist zu empfehlen, wenn ein besonders geringer Druckverlust gewünscht wird.

Weiterhin ist die parallele Anordnung von Rohrreaktoren oder die Verwendung eines Vielrohrreaktors, wobei die Rohre die Funktion der Einbauten übernehmen können, denkbar. Außerdem können Reaktoren mit einer Mehrfacheinspeisung von Gas über die Reaktorlänge versehen werden, wenn der Gasverbrauch so hoch ist, daß ungünstige Phasenverhältnisse von Gas zu Flüssigkeit bei der Zusammenführung der beiden Phasen vor dem Reaktor resultieren.

Die besonderen Bedingungen des erfindungsgemäßen Verfahrens lassen weitere Ausführungsformen des Verfahrens zu. So kann der hohe notwendige Kreislauf der Katalysatorphase bzw. der kontinuierlichen Phase zusätzlich zum Betrieb einer Strahldüse, die als Flüssigkeitsstrahl-Gasverdichter vor dem eigentlichen Rohrreaktor angeordnet wird, ausgenutzt werden. Dies kann zur gründlichen Vorvermischung der beiden Phasen sowie zur Verdichtung der Gasphase, was die Fahrweise bei höheren Vordrücken im Reaktor ermöglicht, eingesetzt werden. Schließlich wird, wenn man umgekehrt statt der Verdichtung des Gases die Saugwirkung ausnutzt, sogar eine Kreislaufführung von Gas unter gleichzeitiger Vorvermischung der Phasen möglich. Die durch die den Katalysator enthaltende kontinuierliche Phase in den Rohrreaktor eingebrachte Energie kann so zur Dispergierung der Eduktphase bzw. mindestens eines Edukts eingesetzt werden.

Bei Reaktionen mit Acetylen, wie bei der Vinylierung von Carbonsäuren, ist die Gefahr eines Acetylenzerfalls bei erhöhtem Druck (ohne Verdünnung) über 1,3 bar absolut gegeben. Dies gilt aber nicht für Acetylen, das fein verteilt in einer Flüssigkeit vorliegt, da dann ein möglicher Zerfall in einer einzelnen Blase sofort durch die umgebende Flüssigkeit gestoppt wird. Da beim Flüssigkeitsstrahl-Gasverdichter die Verdichtung gerade durch das feine Einmischen in den Treibstrahl geschieht, ist hiermit eine höhere Verdichtung von Acetylen gefahrlos möglich. Fällt die Kreislaufpumpe aus, fehlt die Energie für die Verdichtung, der Reaktor läuft leer und das freiwerdende Acetylen ist wieder drucklos. Schließlich wirkt der mit Füllkörpern oder Einbauten versehene Reaktor wie eine Zerfallssperre, so daß eine doppelte Sicherheit gegeben ist.

Auch die Wärmeabfuhr bei stark exothermen Reaktionen wie zum Beispiel bei der Vinylierung ist beim erfindungsgemäßen Verfahren unkritisch. Der hohe Durchsatz des Katalysatorkreislaufs wirkt als Wärmeträger, so daß selbst bei adiabatischer Fahrweise des Reaktors nur geringe Temperaturdifferenzen auftreten und eine homogene Temperaturverteilung im Reaktor ohne Temperaturspitzen resultiert. Die erzeugte Wärme läßt sich dann bequem durch einen beliebig im äußeren Katalysatorkreislauf angeordneten, konventionellen Wärmetauscher abführen oder zur Energiegewinnung ausnutzen. Zur besseren Wärmeabfuhr kann es unter Umständen günstig sein, den Katalysatorkreislauf noch höher zu fahren (also bei einem höheren B-Wert) als nach den Versuchsergebnissen notwendig ist, da über den Katalysatorkreislauf ein kleiner Temperaturgradient über den Reaktor einstellbar ist.

Das erfindungsgemäße Verfahren bietet im Vergleich zum Stand der Technik erhebliche Vorteile, genannt seien:

- Bei vergleichsweise niedrigen Temperaturen können hohe Raum-Zeit-Ausbeuten erreicht werden.
- Die Bildung von Nebenprodukten ist extrem niedrig, Werte von 1 - 2 Gew.-% und darunter sind möglich.
- Der Katalysator wird geschont, die Desaktivierung ist sehr gering, eine kontinuierliche Ausschleusung entfällt.
- Der Umsatz von Acetylen ist sehr hoch und kann 98 % und mehr betragen, so daß eine Acetylenrückführung entfällt. In der Praxis wird man sogar absichtlich eine vom Verfahren an sich nicht notwendige kleine Abgasrate einstellen, da Acetylen Inerte enthält, die sich wegen des hohen Umsatzes anreichern und ausgeschleust werden müssen.
- Durch die besondere Fahrweise wird erreicht, daß trotz des geringen erlaubten Acetylen-Vordrucks von nur 300 mbar auch ein technischer Reaktor mit vielen Metern Länge betrieben werden kann.

Die spezifische Verweilzeit und der Belastungsfaktor sind entsprechend des erfindungsgemäßen Verfahrens zu wählen. Hierzu kann in einen oder mehreren Rohrreaktoren mit Einbauten entsprechend der vorangegangenen Beschreibung gearbeitet werden.

Im erfindungsgemäßen Verfahren, bei der Vinylierung von Carbonsäuren, kann ein Massenverhältnis zwischen Katalysatorphase und Gasphase am Reaktoreingang im Bereich 1000/1 bis 2/1, vorzugsweise im Bereich 200/1 bis 50/1 liegen.

Die Reaktanten können vorgewärmt, d. h. im Bereich der Reaktionstemperatur, oder kalt zugeführt werden. Aufgrund des hohen Phasenverhältnisses mit der Katalysatorphase kann die Vorwärmung auch durch die Prozeßwärme erfolgen.

Die Reaktionswärme kann über verschiedene Wärmeaustauscher abgeführt werden. Die Wärmeaustauscher müssen hierbei nicht in der Nähe des Reaktionsraums sein, sondern können auch beliebig außerhalb des Reaktors liegen. Die einzelnen Wärmeflüsse sind abhängig von der spezifischen Reaktionswärme sowie von den gewünschten Temperaturen im Reaktor und in den Aufarbeitungsvorrichtungen.

Die abgeführte Reaktionswärme kann so sehr einfach genutzt werden, z.B. im Prozess selbst, zur Beheizung einer Destillationseinrichtung oder zur Erzeugung von Dampf.

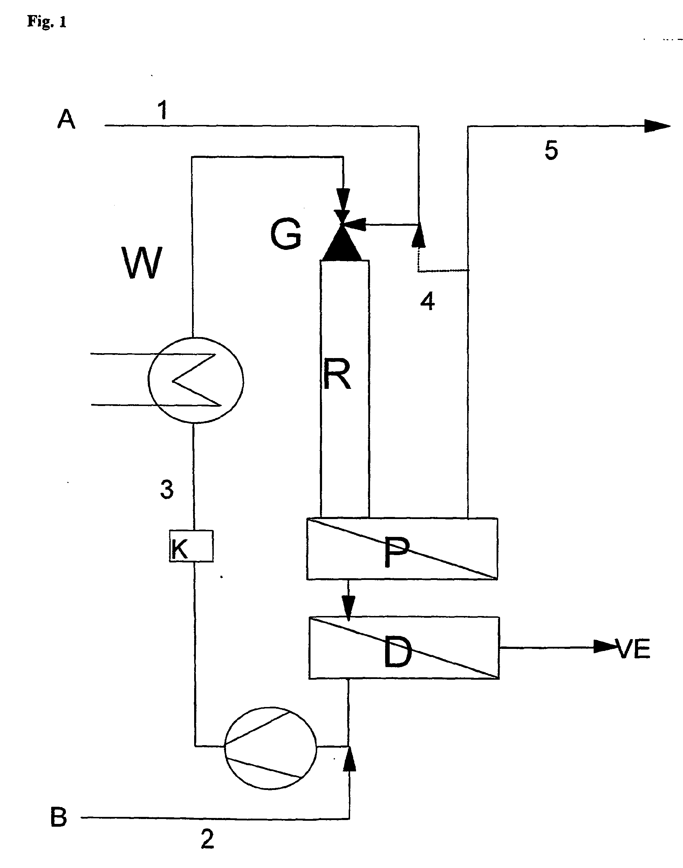

In Fig. 1 ist eine mögliche Ausführungsform des erfindungsgemäßen Verfahrens gezeigt, wobei angenommen wird, daß ein gasförmiges Edukt A mit einem flüssigen Edukt B in Anwesenheit einer flüssigen Katalysatorphase K reagiert. Die Katalysatorphase K wird im Katalysatorkreislauf Strom 3 direkt auf den Kopf des Reaktors R gepumpt, wobei dem Reaktor optional ein Flüssigkeitsstrahl-Gasverdichter G, d. h. eine Strahldüse vorgeschaltet sein kann. Das Einsatzgas in Strom 1 wird über die Saugseite des Flüssigkeits-Gasstrahl-Verdichters (bzw. der Strahldüse) G zugeführt. Wenn dieser nicht vorhanden ist, werden die beiden Ströme einfach ohne weitere Maßnahmen zusammengeführt. Nach erfolgter Reaktion im Reaktor R wird überschüssiges Gas mit üblichen technischen Mitteln abgetrennt (Strom 5), z. B. durch einen Ausgaser oder einen Verweilzeitbehälter P. Die organische Phase geht zur eigentlichen Produkttrennung in den Behälter D. Das Rohprodukt VE kann von der Katalysatorphase z. B. durch Destillation oder Flashen des Reaktoraustrags abgetrennt werden, wenn der Katalysator und das gegebenenfalls verwendete Lösungsmittel höher sieden als das Produkt. Auch andere dem jeweiligen Problem angemessene technische Methoden wie Extraktion, Pervaporation usw. sind möglich, entscheidend ist, daß der Katalysator zurückgewonnen und im Kreise geführt werden kann (Strom 3). Neben der Kreislaufpumpe H ist mit dem Wärmetauscher W die Möglichkeit gezeigt, die Reaktormischung bei endothermen Reaktionen zu heizen bzw. bei exothermen Reaktionen zu kühlen oder die Reaktionswärme, z. B. durch Dampferzeugung anderweitig zu nutzen.

In der Regel wird anschließend an den Behälter D eine Produktreinigung notwendig sein, die wie üblich durch Destillation, Extraktion oder beliebige andere Produktreinigungsverfahren erfolgen kann (nicht dargestellt).

Weiterhin kann das Restgas durch die Verbindung 4 zwischen Strom 5 und Strom 1 im Kreis geführt werden.

Der Reaktor kann auch von unten nach oben oder in einer beliebigen anderen Lage betrieben werden. Beim Betrieb von unten nach oben werden zweckmäßig Gas und Katalysator am Fuß des Reaktors eingespeist, die Ausgaseinheit ist am Kopf des Reaktors angeordnet. Nach der Trennung von Produkt- und Katalysatorphase, wird der Katalysator zum Fuß des Reaktors zurückgeführt.

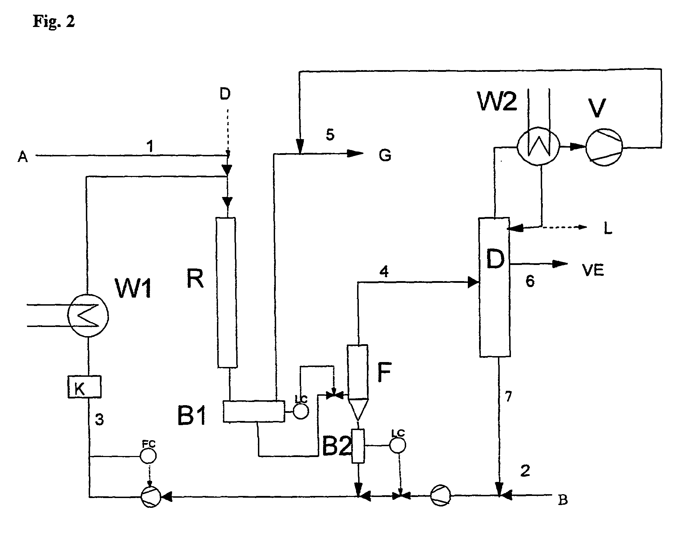

Das Verfahren der vorliegenden Erfindung ist geeignet zur Vinylierung von Carbonsäuren mit Acetylen. Dies soll durch Fig. 2 skizziert werden. Der Flüssigkeitsstrahler (Strahldüse) nach Fig. 1 kann optional hinzugenommen werden. Zweckmäßig wird man den Reaktor von oben nach unten betreiben und für eine dimensionslose Belastung mit B größer 0.8 am Reaktoreingang auslegen. Im Belastungsbereich B = 0,8 bis 1 wird der Druckverlust etwa gleich dem statischen Druck, so daß auf der Gasseite scheinbar kein Druckabfall erfolgt Das Acetylen kann somit drucklos oder nur mit einem geringem Überdruck von maximal 300 mbar zugefahren werden kann. Edukt A (Strom 1) ist in diesem Fall Acetylen, Edukt B (Strom 2) die zu vinylierende Carbonsäure. Der Katalysatorkreislauf (Strom 3) besteht aus einer Lösung des Zinksalzes der zu vinylierenden Säure in überschüssiger Carbonsäure B.

Im erfindungsgemäßen Verfahren kann als Katalysator ein Metallsalz, bevorzugt das Zinksalz der zu vinylierenden Carbonsäure, einsetzt werden.

Der Katalysator wird in überschüssiger Carbonsäure gelöst, so daß im vorliegenden Fall die kontinuierliche Phase durch die Katalysatorlösung und die disperse Phase durch Acetylen gebildet wird.

Als Ausgaser hinter dem eigentlichen Reaktor R dient ein Verweilzeitbehälter B1, der gleichzeitig als Puffervolumen und Arbeitsbehälter ausgelegt werden kann.

Da Vinylester niedriger sieden als die eingesetzte Carbonsäure, kann die Abtrennung vom Katalysator thermisch erfolgen, zum Beispiel durch Destillation. Als besonders günstig, weil produktschonend, hat sich eine Vorabtrennung des Katalysators von Produkten durch einen Flash F erwiesen, wobei die im Flash anfallenden Dämpfe (d. h. gasförmiges Produkt) direkt zu einer Destillationskolonne D geleitet werden, in der die Abtrennung der Vinylester VE (Strom 6) von restlicher Carbonsäure (Strom 7) erfolgt, die bei der einstufigen Trennung im Flashbehälter über den Partialdampfdruck mit ausgetragen wird.

Die am Sumpf anfallende Carbonsäure kann per Strom 7 in die Reaktion zusammen mit frischer Carbonsäure zur Ergänzung des verbrauchten Anteils zurückgeführt werden. Flash F und Kolonne D werden bei Vinylestern mit höherem Molekulargewicht und damit höherem Siedepunkt im Vakuum betrieben, wobei das Vakuum zweckmäßigerweise so einzustellen ist, daß die Siedetemperatur knapp unterhalb der gewünschten Reaktionstemperatur liegt. Hierdurch kann die Reaktionswärme direkt für die Destillation genutzt werden.

Am Kopf der Kolonne D fallen Leichtsieder L an, die in der Regel durch ein gelegentliches Abstreifen des Rücklaufes abgetrennt werden können. Der im Seitenstrom 6 abgezogene Vinylester ist hochrein und kann direkt z. B. für Homo- oder Copolymerisationsreaktionen eingesetzt werden. Zur längeren Lagerung sollte er mit einem Stabilisator versetzt werden, um eine unerwünschte Eigenpolymerisation zu vermeiden.

Am Kopf des Reaktors R kann noch eine Dampfeinspeisung D vorgesehen werden. Hier können geringe Mengen Dampf so eindosiert werden, daß der Katalysator ständig eine geringe Menge Wasser, z.B. 0,1-0,5 Gew.-%, enthält. Dies bremst zwar etwas die Reaktion, verhindert aber die Bildung von Anhydriden der Carbonsäure, die der Reaktion nicht mehr zugänglich wären und als Inerte ausgeschleust werden müßten.

Der Katalysatorkreislauf 3 wird bevorzugt mit konstanter Menge gefahren (FC in Fig. 2), was ein sehr ruhiges und stabiles Verhalten des Reaktors zur Folge hat. Das im Ausgaser B1 anfallende Rohprodukt wird standgeregelt zum Flash F überführt, das hier abgetrennte flüssige Katalysator-Carbonsäuregemisch geht zu einem Vorlagebehälter B2 für die Katalysatorkreislaufpumpe.

In der gesamten Anlage ist das Volumen konstant, außer im Vorlagebehälter B2. Dessen Standabnahme ist ein direktes Maß für den dampfförmig in die Destillationskolonne D abgezogenen Produktanteil, so daß die Standmessung des Behälters B2 direkt die Führungsgröße für den zu ergänzenden Carbonsäureanteil ist.

Die folgenden Beispiele sollen die Erfindung näher beschreiben, ohne deren Anwendungsbreite einzuschränken.

**Beispiel 1 (Vergleichsbeispiel)**

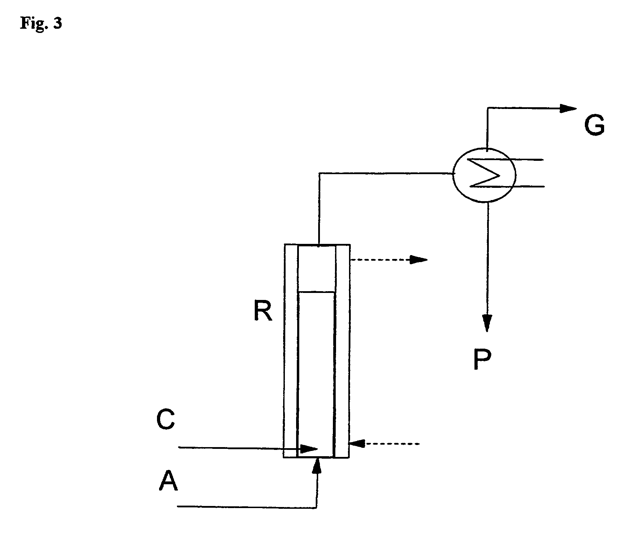

Die Vinylierung erfolgte in einer Versuchsapparatur (s. Fig. 3), wie sie ähnlich in der Literatur beschrieben ist (G. Hübner, Fette, Seifen, Anstrichm. 68, 290 (1966), DE 1210810, DE 1238010). Der Reaktor R hatte eine Höhe von 20 cm, einen Durchmesser von 10 cm und wurde zur Verbesserung des Stoffübergangs mit Raschigringen gefüllt. Über einen Doppelmantel konnte der Reaktor thermostatisiert werden. Acetylen A und die umzusetzende Carbonsäure C wurden am Fuß des Reaktors R über eine Fritte eingespeist.

Der Katalysator bestand aus dem Zinksalz der zu vinylierenden Säure, gelöst in einem Säureüberschuß. Die Reaktion wurde bei einem Gehalt von 55 bis 80 Gew.-% Zinksalz in überschüssiger Säure durchgeführt. Die Reaktorfüllung betrug 1.35 Liter.

Das eingesetzte Acetylen wurde in Abänderung der Patentvorschrift DE 1238010 bei 40 °C statt bei 20 °C mit Wasser gesättigt, sonst verharzte der Katalysator zu schnell. Das Produkt P wurde mit nicht umgesetzter Carbonsäure mit dem Abgas ausgetragen und im Wärmetauscher W kondensiert. Das Abgas G wurde in eine Abgasleitung abgeführt.

Als Carbonsäure wurden tertiäre Nonansäuren mit einer Reinheit von 99,73 %, bezogen auf den Gehalt an tertiären Nonansäuren, mit folgender Zusammensetzung (Angaben in Gew.-%) eingesetzt:

| | |
|---|---|
| 2,2-Dimethyl-Heptansäure | 7,31 |
| 2-Methyl-2-Ethyl-Hexansäure | 54,30 |
| 2-Methyl-2-Propyl-Pentansäure | 7,09 |
| 2,2-Diethyl-Pentansäure | 3,40 |
| 2,2,5-Trimethyl-Hexansäure | 0,83 |
| 2,2,4-Trimethyl-Hexansäure | 0,81 |
| 2,4-Dimethyl-2-Ethyl-Pentansäure | 1,76 |
| 2,2,3-Trimethyl-Hexansäure | 2,54 |
| 2-Methyl-2-Isopropyl-Pentansäure | 5,44 |
| 2,3-Dimethyl-2-Ethyl-Pentansäure A | 7,27 |
| 2,3-Dimethyl-2-Ethyl-Pentansäure B | 7,59 |
| 2-Ethyl-2-Isopropyl-Butansäure | 1,19 |
| Sonstige $C_9$-Säuren | 0,20 |
| unbestimmte Verunreinigungen | 0,27 |

In einer Versuchsreihe wurde die Temperaturabhängigkeit der Raum-Zeit-Ausbeute (RZA) ermittelt.

Bei einer Reaktionstemperatur von 190 °C fand kaum Umsatz statt, erst im Bereich ab 230 °C wurden technisch interessante RZA von 0,3 t/m$^3$/h erzielt. Bei allen Versuchen war ein nur geringer Acetylenumsatz zu verzeichnen, obgleich die Carbonsäure in hohem Überschuß vorhanden war. Es muß mit Kreisgasströmen von Acetylen in der Größenordnung von 100 Mol pro Mol gebildeten Vinylesters bei 190 °C bis (im besten Fall) - 6 Mol pro Mol bei 235 °C gerechnet werden.

Aus Abgasanalysen wurde abgeschätzt, daß bei 235 °C bereits ca. 10 % des umgesetzten Acetylens zu Acetaldehyd reagiert hatten. Daneben wurde im Abgas Benzol, Butenin, Crotonaldehyd und weitere nicht bestimmte Leichtsieder gefunden. Der erforderliche Vordruck des Acetylens lag bei - 0,09 bar/m, so daß ein Reaktor mit dem erlaubten Vordruck von 0,3 bar von höchstens 3 m Höhe betrieben werden könnte, da eine gewisse Druckreserve für den Druckabfall in Leitungen und Regelungen technisch zu berücksichtigen ist.

| Versuch Nr. | 1 | 2 | 3 | 4 | 5 | 6 | 7 | 8 | 9 |
|---|---|---|---|---|---|---|---|---|---|
| Temperatur [°C] | 190 | 210 | 220 | 225 | 231 | 235 | 235 | 235 | 235 |
| Acetylen [Mol/h] | 22,00 | 22,00 | 22,00 | 22,00 | 22,00 | 22,00 | 16,50 | 19,25 | 24,75 |
| Vinylester [Mol/h] | 0,22 | 1,10 | 1,69 | 2,27 | 2,71 | 3,30 | 3,01 | 3,28 | 3,45 |
| Ausbeute an Vinylester bezogen auf Acetylen-Einsatz | 1,0% | 5,0% | 7,7% | 10,3% | 12,3% | 15,0% | 18,2% | 17,0% | 13,9% |
| RZA [t(m$^3$/h] | 0,03 | 0,15 | 0,23 | 0,31 | 0,37 | 0,45 | 0,41 | 0,45 | 0,47 |

**Beispiel 2 (Vergleichsbeispiel)**

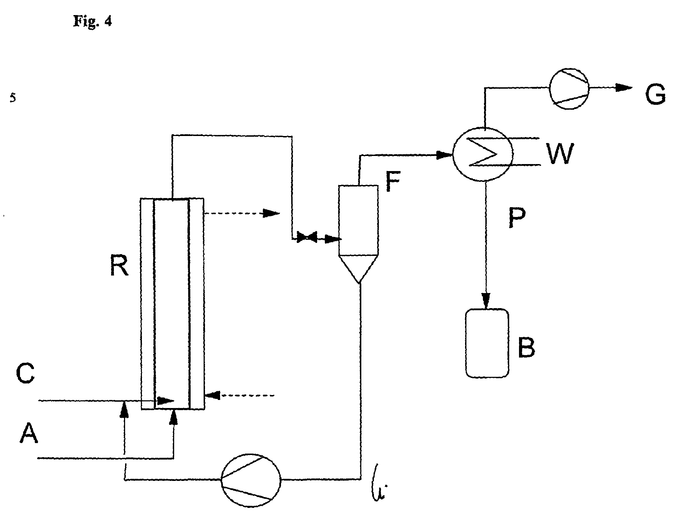

In diesem Versuch wurde gemäß Fig. 4 ein mit Doppelmantel thermostatisierbarer Reaktor R eingesetzt, der zur Verbesserung des Stoffübergangs mit Sulzer-Mischern bestückt war. Der Katalysator und frische Carbonsäure C mit der in Beispiel 1 angegebenen Zusammensetzung wurden dem Reaktor R mit konstant 8 l/h am Fuß zugeführt. Acetylen A wurde mit konstant 4,5 Mol/h über eine Fritte eingespeist. Der Reaktoraustrag wurde nach Druckentspannung über einen Dünnschichtverdampfer F geführt, der bei 80 mbar betrieben wurde, um den gebildeten Vinylester zusammen mit ca. 15 % Carbonsäure abzudestillieren. Dies ist in der Vinylesterbilanz berücksichtigt.
Im Wärmetauscher W wurde das Restgas G entsorgt, die Produktphase P wurde im Behälter B gesammelt.
Der Reaktor wurde unter den typischen Bedingungen einer Blasensäule betrieben und daher nicht unter den Bedingungen des erfindungsgemäßen Verfahrens. (B = 0,27-0,52) Bei sonst konstanten Bedingungen wurde zusätzlich der Einfluß des Wassergehalts im Katalysator untersucht. Dies wurde durch Einspeisung einer geringen Menge Wasserdampf in das zugeführte Acetylen eingestellt und in Proben der Katalysatorlösung durch Karl-Fischer-Titration bestimmt.
An den nämlichen Proben wurden auch analytische Bestimmungen des Zink-Gehalts sowie Dichte- und Viskositätsbestimmungen in Abhängigkeit von der Temperatur durchgeführt. Die entsprechenden Daten für Acetylen wurden aus Tabellen interpoliert.

Apparatekenngrößen:

| Reaktor DN40 | Länge der Füllkörperzone L | 1 m |
| | Durchmesser D | 0,0431 m |
| Füllkörper | SMV 8 DN 40 | Fa. (Sulzer) |
| | Hydraulischer Durchmesser d$_H$ | 0,0072 m |
| | Leerrohranteil | $\Psi$ = 83 % |
| Katalysator | Kreislauf | 0,008 m$^3$/h |
| | Zinksalz/Carbonsäure | 55 - 60 Gew.-% Zn-Salz |

Ergebnisse:

| Versuch Nr. | 10 | 11 | 12 | 13 |
|---|---|---|---|---|
| Temperatur [°C] | 190 | 190 | 190 | 190 |
| Wassergehalt [w-%] | 0,12% | 0,21% | 0,38% | 0,72% |
| Kat.kreislauf [m$^3$/h] | 0,008 | 0,008 | 0,008 | 0,008 |

(fortgesetzt)

| Versuch Nr. | 10 | 11 | 12 | 13 |
|---|---|---|---|---|
| Kat.kreislauf [kg/h] | 7,336 | 7,336 | 7,336 | 7,336 |
| $\rho$ Kat [kg/m$^3$] | 917 | 917 | 917 | 917 |
| $\eta$ Kat [Pa*s] | 1,92E-02 | 1,53E-02 | 9,27E-03 | 2,07E-03 |
| $\rho$ Acetylen [kg/m$^3$] | 0,68 | 0,68 | 0,68 | 0,68 |
| $\eta$ Acetylen [Pa*s] | 1,53E-05 | 1,53E-05 | 1,53E-05 | 1,53E-05 |
| Acetylen ein [Mol/h] | 4,50 | 4,50 | 4,50 | 4,50 |
| Acetylen aus [Mol/h] | 2,16 | 2,72 | 2,97 | 3,36 |
| Acetylen ein [kg/h] | 0,117 | 0,117 | 0,117 | 0,117 |
| Acetylen aus [kg/h] | 0,056 | 0,071 | 0,077 | 0,087 |
| Acetylen ein [m$^3$/h] | 0,171 | 0,171 | 0,171 | 0,171 |
| Acetylen aus [m$^3$/h] | 0,048 | 0,061 | 0,067 | 0,075 |
| Acetylen Umsatz | 52,1% | 39,6% | 34,0% | 25,3% |
| PD[bar] | 2,14E-03 | 1,98E-03 | 1,68E-03 | 1,10E-03 |
| PS [bar] | 4,09E-03 | 4,09E-03 | 4,09E-03 | 4,09E-03 |
| B | 0,52 | 0,49 | 0,41 | 0,27 |
| Vinylester [kg/h] | 0,42 | 0,31 | 0,28 | 0,18 |
| RZA [t/m$^3$/h] | 0,27 | 0,20 | 0,18 | 0,12 |

Es sind immer noch geringe Umsätze des Acetylens zu verzeichnen, obgleich der Nachteil des Beispiels 1, daß das Produkt mit dem Abgas ausgetragen werden muß, durch den Betrieb der Flash-Abtrennung beseitigt ist. Dies erfordert jedoch bei einer großtechnischen Lösung immer noch ein Acetylenkreisgas in der Größenordnung von 2 - 4 Mol/Mol, bezogen auf gebildeten Vinylester. Außerdem liegt der notwendige Vordruck in einem ähnlichen Bereich wie im Beispiel 1, so daß technisch eine maximale Reaktorhöhe von ca. 3 m möglich ist.

**Beispiel 3 (erfindungsgemäßes Verfahren)**

Das erfindungsgemäße Verfahren wurde in einer Technikumsanlage gemäß Figur 2 erprobt, zum Einsatz kam die gleiche tertiäre Nonansäure wie in den Beispielen 1 und 2.

Als Reaktor R diente ein Rohr DN 40, das mit einem Drahtgestrick der Firma VFF (Vereinigte Füllkörper Fabriken, Typ T-20-M) gefüllt war, wie es zur Herstellung von Tropfenabscheidern, sogenannten Demisterpackungen, verwendet wird (Versuche 14, 15, 16). Das Drahtgestrick wurde dichter gerollt als üblich und so in den Reaktor gepackt, daß eine größere Packungsdichte und damit ein kleinerer hydraulischer Durchmesser und ein kleinerer Leerohranteil resultierte. Die Packungslänge betrug 2000 mm. Der Leerrohranteil wurde durch Auslitern des leeren und des gefüllten Reaktors bestimmt, die Packungsdichte durch Wiegen der eingebauten Packung. Die resultierenden Füllkörperkenngrößen sind in den Einzelversuchen angegeben. Dem Reaktor war bei den Versuchen 14, 15 und 16 ein Flüssigkeitsstrahlverdichter vorgeschaltet. Am Fuß des Reaktors konnte eine Ringblende eingebaut werden, die eine Erhöhung des Druckes am Reaktorausgang ermöglichte. Der Druckabfall über die Packungslänge wurde mit einer Differenzdruckmessung erfaßt.

Für Versuch 17 wurde das Drahtgestrick entfernt und durch Mischer der Firma Sulzer (Typ SMV 8 DN 40) ersetzt. Außerdem wurde auf den Flüssigkeitsstrahl-Gasverdichter verzichtet.

Am Kopf des Reaktors wurde der Katalysatorstrom zugeführt, der aus 65 Gew.-% Zinksalz der zu vinylierenden Carbonsäure bestand, gelöst in überschüssiger Säure. Das Acetylen wurde über die Saugseite des Flüssigkeitsstrahlverdichters eindosiert (Versuche 14, 15, 16). Bei Versuch 17 wurde das Acetylen direkt über ein Rohr unmittelbar vor dem Reaktorkopf ohne weitere Vermischungshilfen in den Katalysatorkreislauf zugegeben. Bei diesem Versuch wurde auf jegliche Regelung der Acetylenzufuhr verzichtet. Gemessen wurde lediglich der Verbrauch, der sich beim anstehenden Acetylenvordruck aus der Leitung (ca. 200 mbar) von selbst einstellte.

Bei allen Versuchen wurden in das Acetylen geringe Dampfmengen so eingespeist, daß der Katalysator nach Analysen einen Wassergehalt von 0,25 +/- 0,05 Gew.-% aufwies.

Der Reaktoraustrag wurde im Verweilzeitbehälter B 1 entgast und standgeregelt dem Verdampfer F zugeführt (Flashverdampfung im Vakuum). Die Brüden gelangten zu einer Destillationskolonne D und wurden in Leichtsieder L, die von Zeit zu Zeit aus dem Kolonnenrücklauf abgestreift wurden, Hochsieder (gemäß dem Partialdampfdruck mit Carbonsäure), die am Sumpf anfielen, und den Vinylester der eingesetzten Carbonsäure, der im Seitenstrom 6 abgezogen wurde, aufgetrennt. Kolonne und Flash wurden mit Hilfe eines Vakuumverdichters bei einem Vakuum von 80 - 100 mbar gehalten.

Der flüssige Austrag des Verdampfers ging über den Zwischenbehälter B2 und eine Kreislaufpumpe LC zurück zum Reaktor. Über die Standregelung B2 wurde die Zufuhr von Carbonsäure gesteuert, die aus dem Sumpf der Destillationskolonne bestand, ergänzt durch frische Carbonsäure.

Die Bildung von Nebenprodukten war so gering, daß sie nicht mehr bilanzierbar war. Aus Dauerversuchen über je eine Woche wurde abgeschätzt, daß die gesamte Nebenproduktbildung unter 1 % des Gesamtumsatzes lag.

Auch ein Nachlassen der Katalysatorleistung wurde nicht beobachtet. Offenbar genügte schon das regelmäßige Ziehen von Analysenproben als Ausschleusung von möglicherweise gebildeten Hochsiedern und von anderen störenden Stoffen.

**Versuchsergebnisse:**

| Versuch | lfd. Nr. | - | **14** | **15** | **16** | **17** |
|---|---|---|---|---|---|---|
| Reaktor | Länge | M | 2 | 2 | 2 | 2 |
| | Durchm. | M | 4,30E-02 | 4,30E-02 | 4,30E-02 | 4,30E-02 |
| Füllkörper | Typ | - | T-20-M | T-20-M | T-20-M | SMV8DN40. |
| | Hydr. Durchm. | M | 4,71E-03 | 4,71E-03 | 4,71E-03 | 7,20E-03 |
| | Leerrohranteil | % | 96,7% | 96,7% | 96,7% | 83,0% |
| Betriebsdaten | Temperatur | °C | 190 | 190 | 190 | 191 |
| | Vordruck | bar | 1,46 | 1,9 | 1,73 | 1,04 |
| | Hinterdruck | bar | 1,04 | 1,7 | 1,35 | 1,04 |
| Katalysator | Kreislauf 1) | m$^3$/h | 1,1 | 1,1 | 1,1 | 1,3 |
| | Zinksalz | w-% | 65 +/- 5% | 65 +/- 5% | 65 +/- 5% | 65 +/- 5% |
| Acetylen | Zugang | Mol/h | 17,43 | 21,62 | 21,62 | 10,86 |
| Vinylester | Produktion | Mol/h | 16,50 | 20,82 | 20,11 | 9,33 |
| Acetylen | Überschuß | Mol/Mol | 1,056 | 1,038 | 1,075 | 1,163 |
| Vinylester | RZA | t/m$^3$/h | 1,047 | 1,321 | 1,276 | 0,592 |
| PS ein | M/v*g ein | mbar/m | 63 | 64 | 62 | 69 |
| PS aus | M/v*g aus | mbar/m | 85 | 89 | 87 | 87 |
| PD ein | $P_{lg}$/L ein | mbar/m | 472 | 492 | 492 | 86 |
| PD aus | $P_{lg}$/L aus | mbar/m | 143 | 141 | 141 | 67 |
| Faktor B | Reaktor ein | - | 7,45 | 7,66 | 7,87 | 1,24 |

In Versuch 14 wurde eine außergewöhnlich hohe Raum-Zeit-Ausbeute von über 1 t/m$^3$/h bei einer verfahrenstypisch sehr niedrigen Temperatur von nur 190 °C erzielt. Durch leichtes Anheben des Druckes konnte eine weitere Verbesserung der Raum-Zeit-Ausbeute (Versuche 15, 16) erreicht werden.

Der Acetylenüberschuß war minimal, so daß auf einen Acetylenkreislauf verzichtet werden konnte. Insbesondere bei Versuch 15 war das Abgas nicht mehr meßbar.

Bei Versuch 17 schließlich wurde der Katalysatorkreislauf so eingestellt, daß der gasseitige Differenzdruck über den Reaktor Null war, sich also Druckverlust und statischer Druck aufhoben. Dennoch war der Reaktor geflutet und lief ruhig und störungsfrei. Die sich von selbst einstellende Acetylenaufnahme führte zu einer immer noch sehr hohen Raum-Zeit-Ausbeute von fast 0,6 t/m$^3$/h, so daß ein beliebig langer Reaktor (gegebenenfalls mit einer Mehrfacheinspeisung von Acetylen) problemlos mit dem erlaubten Acetylenvordruck von maximal 300 mbar betrieben werden könnte.

### Beispiel 4 (erfindungsgemäßes Verfahren)

Orientierend wurde in der in Beispiel 3 beschriebenen Versuchsanlage die Vinylierung von 2-Ethyl-Hexansäure (2EHS) untersucht. Ohne weitere Optimierung wurden auch hier schon technisch interessante Raum-Zeit-Ausbeuten von 0,3 t/m$^3$/h erzielt, obgleich diese Säure sehr viel schwieriger vinylierbar ist als eine tertiäre Carbonsäuren.

| Versuch | lfd. Nr. | - | **18** | **19** | **20** |
|---|---|---|---|---|---|
| Carbonsäure | | | 2EHS | 2EHS | 2EHS |
| Reaktor | Länge | m | 2 | 2 | 2 |
| | Durchm. | m | 4,30E-02 | 4,30E-02 | 4,30E-02 |
| Füllkörper | Typ | - | SMV8DN40 | SMV8DN40 | SMV8DN40 |
| | Hydr. Durchm. | m | 7,20E-03 | 7,20E-03 | 7,20E-03 |
| | Leerrohranteil | % | 83,0% | 83,0% | 83,0% |
| Betriebsdat. | Temperatur | °C | 200 | 195 | 190 |
| | Vordruck | bar | 1,114 | 1,114 | 1,114 |
| | Hinterdruck | bar | 1,04 | 1,04 | 1,04 |
| Katalysator | Kreislauf | m$^3$/h | 1,33 | 1,33 | 1,32 |
| | Zinksalz | w-% | 65 +/- 5% | 65 +/- 5% | 65 +/- 5% |
| Acetylen | Zugang | Mol/h | 8,739 | 6,757 | 7,432 |
| Vinylester | Produktion | Mol/h | 5,65 | 3,70 | 4,90 |
| Acetylen | Überschuß | Mol/Mol | 1,548 | 1,825 | 1,518 |
| Vinylester | RZA | t/m$^3$/h | 0,331 | 0,217 | 0,287 |
| PS ein | M/V.g ein | mbar/m | 73 | 76 | 75 |
| PS aus | M/V.g aus | mbar/m | 83 | 86 | 85 |
| PD ein | P$_{lg}$/L ein | mbar/m | 129 | 134 | 140 |
| PD aus | P$_{lg}$/L aus | mbar/m | 106 | 110 | 116 |
| Faktor B | Reaktor ein | - | 1,78 | 1,76 | 1,86 |

### Beispiel 5 (erfindungsgemäßes Verfahren)

Carbonsäuren mit relativ kleinen aliphatischen Ketten, wie z. B. Buttersäure, sind in der Regel der Vinylierung nach dem Zinksalzverfahren nicht zugänglich, da wegen des hohen Dampfdruck der Säuren die notwendigen Reaktionstemperaturen von üblicherweise > 200 °C nicht erreicht werden können. Beispielsweise hat Buttersäure bei Normaldruck von 1013 mbar einen Siedepunkt von 165,5 °C.

Die Versuche wurden wie in den Beispielen 3 und 4 durchgeführt, wobei dem Reaktor ein Flüsigkeitsstrahl-Gasverdichter (Strahldüse) zur Acetyleneinspeisung vorgeschaltet war. Die Reaktionstemperatur wurde so niedrig gewählt, daß sie unter dem Siedepunkt der Zinksalz-Buttersäure-Mischung lag.

In Versuch 21 wurde ein Reaktor verwendet, der mit Maschendrahtringen 6*6 mm gefüllt war. Diese Maschendrahtringe sind in der Destillationstechnik üblich (VFF Vereinigte Füllkörper Fabriken).

Versuchsergebnisse:

| Versuch | lfd. Nr. | - | 21 | 22 | 23 | 24 |
|---|---|---|---|---|---|---|
| Reaktor | Länge | m | 2 | 2 | 2 | 2 |
| | Durchm. | m | 6,40E-02 | 4,30E-02 | 4,30E-02 | 4,30E-02 |
| Füllkörper | Typ | - | MDR 6*6 | SMV8DN 40 | SMV8DN 40 | SMV8DN 40 |
| | Hydr. Durchm. | m | 2,55E-03 | 7,20E-03 | 7,20E-03 | 7,20E-03 |
| | Leerrohranteil | % | 91,5% | 83,0% | 83,0% | 83,0% |
| Betriebsdat. | Temperatur | °C | 180 | 167 | 177 | 177 |
| | Vordruck | bar | 1,06 | 2 | 2 | 2,35 |
| | Hinterdruck | bar | 1,04 | 2,00 | 2 | 2,3 |
| Katalysator | Kreislauf 1) | $m^3$/h | 1,04 | 1,59 | 1,26 | 1,26 |
| | Zinksalz | w-% | 50,0% | 55,0% | 84,2% | 84,2% |
| Acetylen | Zugang | Mol/h | 5,14 | 6,31 | 10,405 | 11,937 |
| Vinylester | Produktion | Mol/h | 1,52 | 1,91 | 4,20 | 5,34 |
| Acetylen | Überschuß | Mol/Mol | 3,37 | 3,30 | 2,48 | 2,23 |
| Vinylester | RZA | t/$m^3$/h | 0,027 | 0,075 | 0,17 | 0,21 |
| PS ein | M/V.g ein | mbar/m | 88 | 97 | 90 | 90 |
| PS aus | M/V.g aus | mbar/m | 99 | 103 | 99 | 100 |
| PD ein | $P_{lg}$/L ein | mbar/m | 239 | 146 | 99 | 99 |
| PD aus | $P_{lg}$/L aus | mbar/m | 113 | 119 | 78 | 78 |
| Faktor B | Reaktor ein | - | 2,72 | 1,50 | 1,10 | 1,10 |

Im Versuch 21 (drucklos) wird eine geringfügige Bildung von Vinylester beobachtet. Durch Drucksteigerung, Anhebung des Zink-Salz-Gehaltes im Katalysator und mäßigen Acetylenüberschuß (Kreisgasfahrweise) wird aber wieder eine deutliche Erhöhung der Raum-Zeit-Ausbeute erreicht. Im Versuch 24 ist mit 0,21 t/$m^3$/h schon eine technisch vernünftige Größenordnung erreicht. Somit sind durch das erfindungsgemäße Verfahren auch niedrige Carbonsäuren wie Buttersäure der Vinylierung zugänglich.

**Patentansprüche**

1. Verfahren zur katalytischen Durchführung von Mehrphasenreaktionen in einem Rohrreaktor
**dadurch gekennzeichnet,**
**daß** der Katalysator in der kontinuierlichen Phase und mindestens ein Edukt in einer dispergierten Phase enthalten ist und der Belastungsfaktor B des Rohrreaktors gleich oder größer 0,8 ist und daß durch die Mehrphasenreaktion Carbonsäuren mit Acetylen zu den entsprechenden Vinylestern umgesetzt werden.

2. Verfahren nach Anspruch 1,
**dadurch gekennzeichnet,**
**daß** die Carbonsäure 2 bis 16 Kohlenstoffatome enthält.

3. Verfahren nach den Ansprüchen 1 oder 2,
**dadurch gekennzeichnet,**
**daß** der Katalysator ein Metallsalz der Carbonsäure ist.

4. Verfahren nach einem der Ansprüche 1 bis 3,
**dadurch gekennzeichnet,**
**daß** der Katalysator das Zinksalz der Carbonsäure ist.

**5.** Verfahren nach einem der Ansprüche 1 bis 4
**dadurch gekennzeichnet,**
**daß** der Belastungsfaktor B größer oder gleich 0,9 ist.

**6.** Verfahren nach einem der Ansprüche 1 bis 5
**dadurch gekennzeichnet,**
**daß** der Belastungsfaktor B größer oder gleich 1,0 ist.

**7.** Verfahren nach einem der Ansprüche 1 bis 6
**dadurch gekennzeichnet,**
**daß** das Massenverhältnis der kontinuierlichen Phase zu der oder den dispersen Phasen größer als 2 ist.

**8.** Verfahren nach einem der Ansprüche 1 bis 7,
**dadurch gekennzeichnet,**
**daß** die kontinuierliche Phase vor dem Rohrreaktor eine Strahldüse antreibt.

**9.** Verfahren nach einem der Ansprüche 1 bis 8,
**dadurch gekennzeichnet,**
**daß** mindestens ein Edukt durch die von der kontinuierlichen Phase in den Rohrreaktor eingebrachte Energie dispergiert wird.

**Claims**

**1.** Process for carrying out catalytic multiphase reactions in a tube reactor, **characterized in that** the catalyst is present in the continuous phase and at least one starting material is present in a dispersed phase and the loading factor B of the tube reactor is equal to or greater than 0.8 and **in that** carboxylic acids are reacted with acetylene in the multiphase reaction to give the corresponding vinyl esters.

**2.** Process according to Claim 1, **characterized in that** the carboxylic acid contains from 2 to 16 carbon atoms.

**3.** Process according to Claim 1 or 2, **characterized in that** the catalyst is a metal salt of the carboxylic acid.

**4.** Process according to any of Claims 1 to 3, **characterized in that** the catalyst is the zinc salt of the carboxylic acid.

**5.** Process according to any of Claims 1 to 4, **characterized in that** the loading factor B is greater than or equal to 0.9.

**6.** Process according to any of Claims 1 to 5, **characterized in that** the loading factor B is greater than or equal to 1.0.

**7.** Process according to any of Claims 1 to 6, **characterized in that** the mass ratio of the continuous phase to the disperse phase or phases is greater than 2.

**8.** Process according to any of Claims 1 to 7, **characterized in that** the continuous phase drives a jet nozzle upstream of the tube reactor.

**9.** Process according to any of Claims 1 to 8, **characterized in that** at least one starting material is dispersed by means of the energy introduced into the tube reactor by the continuous phase.

**Revendications**

**1.** Procédé pour la réalisation catalytique de réactions à plusieurs phases dans un réacteur tubulaire, **caractérisé en ce que** le catalyseur est contenu dans la phase continue et au moins un produit de départ est contenu dans une phase dispersée et le facteur de charge B du réacteur tubulaire est égal ou supérieur à 0,8 et **en ce que** la réaction à plusieurs phases sert à transformer des acides carboxyliques avec de l'acétylène en esters de vinyle correspondants.

**2.** Procédé selon la revendication 1, **caractérisé en ce que** les acides carboxyliques contiennent 2 à 16 atomes de carbone.

3. Procédé selon les revendications 1 ou 2, **caractérisé en ce que** le catalyseur est un sel métallique de l'acide carboxylique.

4. Procédé selon l'une quelconque des revendications 1 à 3, **caractérisé en ce que** le catalyseur est un sel zincique de l'acide carboxylique.

5. Procédé selon l'une quelconque des revendications 1 à 4, **caractérisé en ce que** le facteur de charge B est supérieur ou égal à 0,9.

6. Procédé selon l'une quelconque des revendications 1 à 5, **caractérisé en ce que** le facteur de charge B est supérieur ou égal à 1,0.

7. Procédé selon l'une quelconque des revendications 1 à 6, **caractérisé en ce que** le rapport massique de la phase continue à la ou aux phases dispersées est supérieur à 2.

8. Procédé selon l'une quelconque des revendications 1 à 7, **caractérisé en ce que** la phase continue entraîne un pulvérisateur en amont du réacteur tubulaire.

9. Procédé selon l'une quelconque des revendications 1 à 8, **caractérisé en ce qu'**au moins un produit de départ est dispersé par l'énergie introduite par la phase continue dans le réacteur tubulaire.

**Fig. 1**

Fig. 2

**Fig. 3**

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 1238010
- DE 1210810
- DE 1237557
- EP 0622352 A
- US 5430179 A

**In der Beschreibung aufgeführte Nicht-Patentliteratur**

- **G. HÜBNER.** *Fette, Seifen, Anstrichm.,* 1966, vol. 68, 290
- Erweiterte. VDI-Verlag GmbH, 1994
- Standardwerk Heinz Brauer, Grundlagen der Ein-phasen- und Mehrphasenströmungen. Verlag Sau-erländer, 1971
- **S. ERGUN.** *Chem. Engng. Progr.,* 1948, vol. 48, 89
- **Y. SATO ; T.HIROSE ; F. TAKAHASHI ; M. TODA.** Pressure Loss and Liquid Hold Up in Packed Bed Reactor with Cocurrent Gas-Liquid Down Flow. *J. Chem. Chem. Eng. Of Japan,* 1973, vol. 6 (2), 147-152
- **D. SWEENEY.** A Correlation for Pressure Drop in Two-Phase Concurrent Flow in Packed Beds. *AIChE-Journal,* Juli 1967, vol. 13, 663-669
- **V. W. WEEKMAN ; J. E. MYERS.** Fluid-Flow Characteristics of Concurrent Gas-Liquid Flow in Packed Beds. *AIChE-Journal,* November 1964, vol. 10 (6), 951-957
- **R. P. LARKINS ; R. P. WHITE ; D. W. JEFFREY.** Two-Phase Concurrent Flow in Packed Beds. *AIChE-Journal,* Juni 1961, vol. 7 (2), 231-239
- **N. MIDOUX ; M. FAVIER ; J.- C. CHARPENTIER.** Flow Pattern, Pressure Loss and Liquid Holdup Data in Gas-Liquid Down-Flow Packed Beds with Foaming and Nonfoaming Liquids. *J. Chem. Eng. Of Japan,* 1976, vol. 9 (5), 350-356